# EUROPEAN PATENT APPLICATION

(11) **EP 4 019 087 A1**
(43) Date of publication of application: **29.06.2022**
(21) Application number: 20854993.1
(22) Date of filing: 11.08.2020
(51) Int. Cl.: A61P 1/00, A61K 33/243

(54) **AGENT FOR REDUCING MALODOR OF FLATULENCE AND/OR STOOL**

(30) Priority: 20.08.2019 JP 2019162429
(71) Applicant: Miyamoto, Yusei, Tokyo 154-0002 (JP)
(72) Inventor: Miyamoto, Yusei, Tokyo 154-0002 (JP)
(74) Representative: Peters, Hajo
(86) International application number: PCT/JP2020/030546
(87) International publication number: WO 2021/033590

(57) **Abstract**

Targeted at, for example, mammalian animals including human, and for the purpose of realizing an effective reduction in the malodor inherent to flatulence and/or stool with an orally ingestible material such as an inorganic substance, provided is an orally ingestible malodor reducing agent for reducing the malodor of flatulence and/or stool, the agent containing nanosized colloidal platinum microparticles as an active ingredient.

## Description

### TECHNICAL FIELD

The present invention relates to a malodor reducing agent for effectively reducing the malodor inherent to flatulence and/or stool.

### BACKGROUND ART

Flatulence and stool of, for example, mammalian animals including human have a unique malodor causing a strong unpleasant feeling to human; desired is a method for reducing such malodor from the perspectives of, for example, beauty, ease in nursing care, or ease in rearing pets in a sanitary condition. This malodor is assumed to be caused by sulfides (hydrogen sulfide, sulfur dioxide, hydrocarbon sulfide), indole, skatole, zinc, ammonia or the like; for example, there is known a method for reducing the malodor of stool by reducing ammonia, indole or the like with the aid of bifidobacteria (Non-patent document 1). However, other than bifidobacteria, there has never been a method for effectively reducing the malodor inherent to flatulence and stool with an orally ingestible material such as an inorganic substance.

There has been an invention for canceling out stool odor or the like by spraying a fragrance to ambient air (Patent document 1); while the champignon essence as a functional food material extracted from mushrooms used to be considered as effective in erasing breath odor, body odor and stool odor, the basis thereof was never recognized, and an exclusion order was issued by Japan Fair Trade Commission in 2009.

In view of these circumstances, it is desired that there be provided a means that enables safe oral administration, and is capable of effectively reducing the malodor of flatulence and stool of, for example, mammalian animals including human.

Meanwhile, there are known, for example, a superoxide anion decomposition agent containing nanosized platinum microparticles (Patent document 2), and an oral cavity cleansing agent containing such platinum microparticles (Patent document 3); it has never been known that platinum microparticles are able to effectively reduce the malodor inherent to flatulence and/or stool.

### PRIOR ART DOCUMENTS

### Patent documents

Patent document 1: Japanese Unexamined Patent Application Publication No. 2018-130565
Patent document 2: Japanese Patent No. 4058072
Patent document 3: WO2006/064788A1

### Non-patent documents

Non-patent document 1: Ogata T., et al, Microbial Ecology in Health and Disease 11:41 (1999)

### SUMMARY OF THE INVENTION

### Problems to be solved by the invention

The object of the present invention is to realize an effective reduction in the malodor inherent to flatulence and/or stool with an orally ingestible material such as an inorganic substance, targeting at, for example, mammalian animals including human.

### Means to solve the problems

The inventor of the present invention surprisingly found that the malodor inherent to flatulence and stool could be effectively reduced by orally ingesting platinum microparticles.

The present invention provides an orally ingestible malodor reducing agent for reducing the malodor of flatulence and/or stool, the agent containing platinum microparticles as an active ingredient.

The malodor reducing agent of the present invention may be a malodor reducing agent for reducing hydrogen sulfide in flatulence and/or stool.

Further, the malodor reducing agent of the present invention may be a malodor reducing agent containing nanosized colloidal platinum microparticles as an active ingredient.

Further, the malodor reducing agent of the present invention may be a malodor reducing agent in which the platinum microparticles have an average particle diameter of not larger than 10 nm.

Further, the malodor reducing agent of the present invention may be a malodor reducing agent containing 1 nmole to 100 mmole of the platinum microparticles per 1,000 ml of water.

Further, the present invention provides a method for reducing the malodor of flatulence and/or stool by orally ingesting platinum microparticles as an active ingredient.

Further, the present invention provides platinum microparticles for use in a method for reducing the malodor of flatulence and/or stool.

The platinum microparticles of the present invention may be platinum microparticles for use in a malodor reducing agent for reducing hydrogen sulfide in flatulence and/or stool.

Further, the platinum microparticles of the present invention may be platinum microparticles used as a malodor reducing agent containing nanosized colloidal platinum microparticles as an active ingredient.

Further, the platinum microparticles of the present invention may be platinum microparticles used as a malodor reducing agent in which the platinum microparticles have an average particle diameter of not larger than 10 nm.

Further, the platinum microparticles of the present invention may be platinum microparticles used as a malodor reducing agent containing 1 nmole to 100 mmole of the platinum microparticles per 1,000 ml of water.

Further, the present invention provides a use of platinum microparticles in the production of a malodor reducing agent.

### Effects of the invention

The malodor reducing agent of the present invention is safe because the agent can be orally ingested by, for example, mammalian animals including human, and almost a full dosage thereof will then be directly egested without being absorbed into the body. For example, the agent can be taken as a suspension containing nanosized platinum microparticles reduced by citric acid and directly employing such citric acid as a protective agent, thereby making it possible to effectively deodorize the malodor inherent to flatulence and/or stool.

### MODE FOR CARRYING OUT THE INVENTION

The present invention is an orally ingestible agent for reducing the malodor of flatulence and/or stool, which contains platinum microparticles as an active ingredient.

In this specification, "platinum microparticles" refer to microparticles of platinum, and include powdery platinum microparticles such as platinum black; platinum microparticles using a protective agent; platinum microparticles not using a protective agent, but using a surfactant; platinum microparticles using neither a protective agent nor a surfactant; and single atom particles.

There are known various methods for producing platinum microparticles (e.g. Japanese Examined Patent Application Publication No. Sho 57-43125, Japanese Examined Patent Application Publication No. Sho 59-120249, Japanese Unexamined Patent Application Publication No. Hei 9-225317, Japanese Unexamined Patent Application Publication No. Hei 10-176207, Japanese Unexamined Patent Application Publication No. 2001-79382 and Japanese Unexamined Patent Application Publication No. 2001-122723); those skilled in the art can easily prepare microparticles by referring to these methods. For example, as a method for producing noble metal microparticles, there can be utilized, for example, a chemical method called precipitation method or metal salt reduction reaction method, or a physical method called combustion method. There may also be employed a liquid phase laser ablation method; and a method for conducting suspension using a surfactant, but without using a protective agent. As the malodor reducing agent of the present invention, there may be used microparticles prepared by any of these methods. A method of microparticulation is, for example, described in the specification of Japanese Patent No. 4058072; the method is thus included in the disclosure of this specification by referring to the disclosure of the captioned publication.

Further, for example, in the case of platinum microparticles using a protective agent, platinum nanoparticles can be uniformly dispersed in water, using a metal protective agent as a substance for protecting fine metal particles so that they will not aggregate. Examples of a protective agent for fine metal particles include polyvinylpyrrolidone, polyacrylic acid, pectin and citric acid; pectin and citric acid are preferred as they are food ingredients.

Further, while the platinum in the malodor reducing agent of the present invention may also be a platinum-containing alloy, preferred is platinum itself. As a metal(s) forming the alloy, there can be listed, for example, gold, ruthenium, rhodium, palladium, osmium and iridium. Together with the platinum microparticles, there may also be used a colloidal microparticle suspended substance of multiple kinds of metals including microparticles of one or more kinds of the above-listed other metals.

As the platinum microparticles, preferred are microparticles having a large specific surface area, a superior surface reactivity, and a capability of forming a colloidal state. For example, the microparticles may be those having a particle diameter of a micrometer level, or those having a particle diameter of a nano level. There are no particular restrictions on the particle diameter of the microparticles; there may be used microparticles having an average particle diameter of not larger than 50 nm, preferably not larger than 20 nm, more preferably not larger than 10 nm, particularly preferably about 1 to 6 nm. Also preferred is a dispersion liquid with these microparticles being contained in an aqueous medium in a stably suspended state. As the aqueous medium, other than water, there may be used an organic solvent that has a low toxicity to a living body and is capable of being mixed with water at any ratio, such as ethanol, ethylene glycol or the like. Preferably, water may be used as the aqueous medium.

It is preferred that the malodor reducing agent of the present invention be prepared in such a manner that, for example, about 1 nmole to 100 mmole, more preferably about 10 nmole to 50 mmole of the platinum microparticles are to be contained in a colloidal state per 1,000 ml of water. A platinum concentration is preferably about 100 to 300 ppm, particularly preferably about 200 ppm.

The malodor reducing agent containing the colloidal platinum microparticles at the above concentration can be orally administered to a mammalian animal including human. For example, by orally ingesting per day about 50 to 5,000 µl, preferably about 200 µl of the malodor reducing agent containing 200 ppm of the colloidal platinum microparticles, the malodor of flatulence and/or stool egested will be remarkably reduced due to the onset of a desired effect in the intestine. That is, an orally ingested dose of the platinum microparticles of the present invention is equivalent to 1×10⁻³ g at maximum per day.

As for the safety of platinum microparticles, there is a report on hepatopathy when administered intravenously (Y. Yagmagishi, A. et al., Pharmazie 68:178-182, 2013). Specifically, 24 hours after intravenously administering platinum nanoparticles having a particle diameter of not larger than 1 nm and platinum nanoparticles having a particle diameter of 15 nm (sub-nanosized platinum particles; respectively referred to as snPt, nPt hereunder) to a mouse, as a result of evaluating the existence or non-existence of hepatopathy by measuring alanine aminotransferase (alanine transaminase; ALT) and aspartate aminotransferase (aspartate transaminase) that had leaked into the blood, hepatopathy was observed only in the case where snPt was administered in a dose of 15 to 20 mg/kg. Meanwhile, in the case of nPt having the particle diameter of 15 nm, hepatopathy was not observed even when administered in the same dose. This intravenous administration in the dose of 15 to 20 mg/kg is equivalent to about 1 g in the case of a human weighing 60 kg. In order for such amount of platinum microparticles to enter the body via oral ingestion, an amount of 1,000 g needs to be orally ingested given that a rate of absorption from the small intestine is 0.1% at maximum (it is indicated in the following working examples that 99.96% of the platinum microparticles of the present invention were able to be recovered in stool if orally ingested by human.). Thus, it can be understood that the oral ingestion dose of the malodor reducing agent of the present invention is highly safe.

The malodor reducing agent of the present invention can be drunk as a health food, and can also be used as a medicine itself.

As a health food, for example, the malodor reducing agent containing 200 ppm of the colloidal platinum microparticles may be added to teas, juices, soft drinks and drinks, and then drunk in these forms.

If using the malodor reducing agent of the present invention as a medicine, the malodor reducing agent may not only be used as an oral liquid agent containing 200 ppm of the colloidal platinum microparticles, but also be produced as a medicinal preparation in the form of a solid preparation such as tablets, capsules, granules and powdered medicines by adding pharmaceutically allowable medicinal additives, and then administered to a patient.

As the pharmaceutically allowable medicinal additives used to produce the medicinal preparation, there may be added, for example, additives known to those skilled in the art, such as a stabilizer, antioxidant, pH adjuster, buffering agent, suspension agent, emulsifier and surfactant so as to be able to produce the medicinal preparation. The types, administrations and dosages of these medicinal additives are described in, for example, Japanese Pharmaceutical Excipients Directory 2016 (edited by IPEC Japan, published by Yakuji Nippo, Limited, February 2016); the medicinal preparation can be produced and used in accordance with these descriptions.

### WORKING EXAMPLES

### 1. Production of platinum nanoparticles

Platinum nanoparticles were produced by a citric acid reduction method. Here, 4 ml of a 16.6 mM chloroplatinic acid (H₂PtCl₆) was added to 43.8 ml of water, followed by performing heating in an oil bath (oil bath). At a time point when the temperature reached 100°C, 8.6 ml of a 77.21 mM trisodium citrate (trisodium citrate; C₆H₅Na₃O₇) was added, and heating was directly continued at 100°C for about 1 hour and 30 min until the reaction solution had turned black. Heating was stopped once the reaction solution had turned black; the temperature of the reaction solution was then lowered to room temperature, and 10 ml of water was added thereto in the end, thereby obtaining 66.4 ml of a platinum nanocolloid liquid having a platinum concentration of 1 mM. The particle diameter of the platinum microparticles was 4.7±1.5 nm.

### 2. Evaluation on effect of reducing odor of flatulence and stool by platinum nanoparticles

Nanosized platinum microparticles were produced; after being orally ingested, the platinum metal was then recovered from stool, and a reduction in the malodor inherent to flatulence and stool was quantified.

As a platinum nanocolloid, there was used a platinum nanocolloid water as a liquid for oral ingestion that contained per 1 ml, as platinum, 180 µg of platinum microparticles having an average particle diameter of not larger than 6 nm, and substantially contained no aggregated platinum particles. There were contained 0.9560% of the platinum nanocolloid (platinum; 0.00019%, trisodium citrate 0.0028%), 0.0045% of citric acid and 99.0395% of water; a platinum content was 172±1.5 ppm, and pH was 3.71.

There is ensured such a safety that when ingested orally, the nanosized platinum microparticles (4.7±1.5 nm) reduced by citric acid and directly employing such citric acid as a protective agent are barely absorbed from digestive canal before arriving at the large intestine, and 99.96% of them will then be egested into stool. The malodor of flatulence and stool before orally ingesting the nanosized platinum metal particles; and the malodor of flatulence and stool three weeks after starting to orally ingest the nanosized platinum metal particles were quantified and compared using a monitor GT300-VOC (distributor: MK Scientific, Inc.) capable of sensitively detecting volatile organic compounds (Volatile Organic Compounds; VOC), smokes, odors or the like, particularly ammonia, toluene and hydrogen sulfide, and displaying a quantified value(s) via CIAQ (Composite Index of Air Quality) where changes in the odors in the air are collectively evaluated with a fresh air being 1. As a result of orally ingesting the nanosized platinum microparticles at a ratio of 40 µg/day per a human body weight of 60 kg for three weeks, the numerical value measured by the odor monitor significantly decreased at a risk rate (p) of lower than 0.01% after orally ingesting the nanosized platinum metal particles for three weeks; it was indicated that the malodor of flatulence and stool had been statistically significantly reduced by orally ingesting the nanosized platinum microparticles (Table 1). Here, the statistical analysis was conducted using Student's t-test.

**[Table 1]**

| | Experimental group | Measured value | Statistically significant difference |
|---|---|---|---|
| Flatulence odor | Before oral ingestion | 79.3 ± 64.0 (n=27) | p < 0.01 |
| | Three weeks after starting oral ingestion | 10.9 ± 10.2 (n=12) | |
| Stool odor | Before oral ingestion | 7.43 ± 1.18 (n=7) | p < 0.01 |
| | Three weeks after starting oral ingestion | 0.57 ± 0.49 (n=7) | |

### 3. Evaluation on effect of reducing hydrogen sulfide in stool

A gas sampling pump kit (model GV100S) manufactured by GASTEC CORPORATION was used to measure, quantify and compare ammonia and hydrogen sulfide dispersed from stool before and after orally ingesting the nanosized platinum microparticles.

Specifically, 100 to 200 g of human stool was weighed out and put into an enamel bedpan, followed by swiftly covering the same with Press'n Seal (by The Glad Products Company) so as to keep maintaining an airtightness; about 3 min later, the concentrations (ppm) of the gases held over the bedpan were quantified using corresponding detecting tubes.

As a result of conducting measurement after a week or later since starting to orally ingest the nanosized platinum microparticles at the ratio of 40 µg/day per the human body weight of 60 kg, while no changes were observed in the ammonia concentration, the hydrogen sulfide concentration had statistically significantly decreased at a risk rate (p) of lower than 0.01%; it was indicated that by orally ingesting the nanosized platinum microparticles, the malodor of stool was able to be reduced at least through a reduction in hydrogen sulfide (Table 2). Here, the statistical analysis was conducted using Student's t-test.

**[Table 2]**

| Measured results of concentrations of ammonia and hydrogen sulfide dispersed from stool | | |
|---|---|---|
| | Ammonia (ppm/g) | Hydrogen sulfide (ppm/g) |
| Before oral ingestion | 1.69 ± 0.48 (n=6) | 5.88± 0.96 (n=6) |
| After oral ingestion | 2.47 ± 1.16 (n=9) | ^{∗∗}1.77 ± 0.69 (n=10) |

| | | |
|---|---|---|
| ^{∗∗}p< 0.01 | | |

## Claims

1. An orally ingestible malodor reducing agent for reducing the malodor of flatulence and/or stool, containing platinum microparticles as an active ingredient.

2. The malodor reducing agent according to claim 1 for reducing hydrogen sulfide in flatulence and/or stool.

3. The malodor reducing agent according to claim 1 or 2, containing nanosized colloidal platinum microparticles as an active ingredient.

4. The malodor reducing agent according to any one of claims 1 to 3, wherein the microparticles have an average particle diameter of not larger than 10 nm.

5. The malodor reducing agent according to any one of claims 1 to 4, containing 1 nmole to 100 mmole of the platinum microparticles per 1,000 ml of water.
